(19) Europäisches Patentamt
European Patent Office
Office européen des brevets

(11) EP 4 559 567 A1

(12) **EUROPEAN PATENT APPLICATION**
published in accordance with Art. 153(4) EPC

(43) Date of publication:
**28.05.2025 Bulletin 2025/22**

(21) Application number: **23842957.5**

(22) Date of filing: **18.07.2023**

(51) International Patent Classification (IPC):
***B01D 69/00*** *(2006.01)* ***B01D 69/08*** *(2006.01)*
***B01D 71/44*** *(2006.01)* ***B01D 71/68*** *(2006.01)*
***A61M 1/16*** *(2006.01)*

(52) Cooperative Patent Classification (CPC):
**A61M 1/16; B01D 69/00; B01D 69/08; B01D 71/44; B01D 71/68**

(86) International application number:
**PCT/JP2023/026206**

(87) International publication number:
**WO 2024/019034 (25.01.2024 Gazette 2024/04)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **20.07.2022 JP 2022115694**

(71) Applicant: **Toyobo Co., Ltd.**
**Kita-ku**
**Osaka-shi, Osaka 530-0001 (JP)**

(72) Inventors:
- **MINEYAMA, Mizuki**
  **Otsu-shi, Shiga 520-0292 (JP)**
- **YAO, Miyuki**
  **Otsu-shi, Shiga 520-0292 (JP)**

(74) Representative: **Vossius & Partner Patentanwälte Rechtsanwälte mbB**
**Siebertstrasse 3**
**81675 München (DE)**

(54) **HOLLOW FIBER MEMBRANE AND PLASMA SEPARATION METHOD**

(57) A hollow fiber membrane comprising polyethersulfone, wherein the hollow fiber membrane has a structure that is non-uniform in a thickness direction, and a gradient index which is a ratio of an average pore size at an inner surface side in a cross section of the hollow fiber membrane to an average pore size at an outer surface side in the cross section is 2.00 or more.

FIG.1

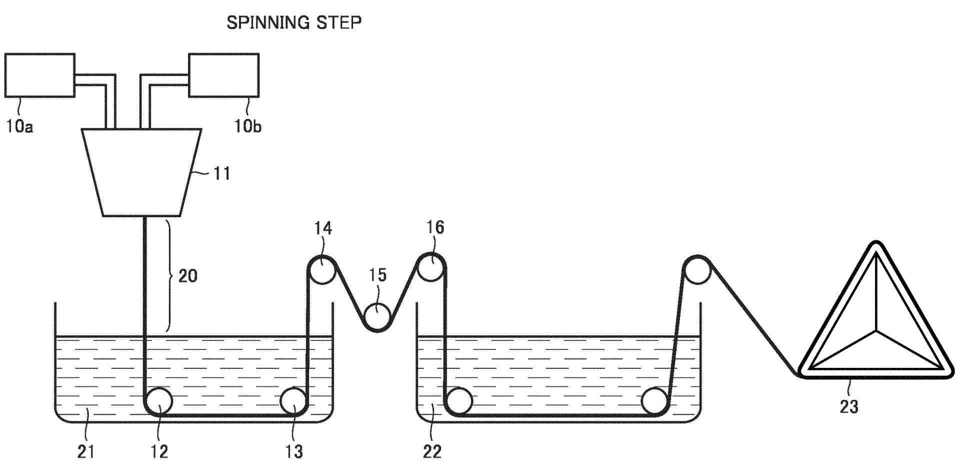

## Description

### TECHNICAL FIELD

The present invention relates to a hollow fiber membrane and a plasmapheresis method.

### BACKGROUND ART

In recent years, devices have been developed for apheresis treatment, which is a procedure to remove disease-causing substances and excess water from blood or body-cavity fluid taken from patients by means of such principles as dialysis, filtration, and adsorption and then return them back into the body of the patients. In particular, plasmapheresis therapy is known, which is a therapy to separate blood that is taken from patients who suffer from hepatic failure, blood diseases, autoimmune diseases, and the like, into blood cells/platelets and plasma by means of a hollow fiber membrane, discard plasma that contains disease-causing substances (such as IgG and cytokines), and supply freshly-frozen plasma and the like.

For example, PTL 1 (Japanese Patent No. 5290168) and PTL 2 (International Patent Laying-Open No. WO 2020/203716) each discloses a hollow fiber membrane (such as a hollow fiber membrane made of polyethersulfone, for example) for use in plasma separation from blood (plasmapheresis) as mentioned above. Other known materials suitable for hollow fiber membranes for plasmapheresis and the like include polyethylene, for example.

### CITATION LIST

### PATENT LITERATURE

PTL 1: Japanese Patent No. 5290168
PTL 2: International Patent Laying-Open No. WO 2020/203716

### SUMMARY OF INVENTION

### TECHNICAL PROBLEM

When a hollow fiber membrane of this type (a hollow fiber membrane made of polyethersulfone) is used for plasmapheresis, clogging of the hollow fiber membrane tends to occur, so there has been a demand for a hollow fiber membrane that can be used in a continuous and stable manner with less clogging.

Accordingly, the present invention aims at reducing clogging of a hollow fiber membrane when the hollow fiber membrane is used in plasmapheresis.

### SOLUTION TO PROBLEM

(1) A hollow fiber membrane comprising polyethersulfone, wherein

the hollow fiber membrane has a structure that is non-uniform in a thickness direction, and
a gradient index which is a ratio of an average pore size at an inner surface side in a cross section of the hollow fiber membrane to an average pore size at an outer surface side in the cross section is 2.00 or more.

(2) The hollow fiber membrane according to (1), wherein a most-frequent pore size in a cross section of the hollow fiber membrane is 0.090 μm or more.
(3) The hollow fiber membrane according to (1) or (2) further comprising a vinylpyrrolidone-based polymer.
(4) The hollow fiber membrane according to any one of (1) to (3) for use in plasmapheresis.
(5) A plasmapheresis method using the hollow fiber membrane according to any one of (1) to (4).

### ADVANTAGEOUS EFFECTS OF INVENTION

The present invention makes it possible to reduce clogging of a hollow fiber membrane made of polyethersulfone

when the hollow fiber membrane is used in plasmapheresis.

BRIEF DESCRIPTION OF DRAWINGS

Fig. 1 is a schematic view for explaining an example of a method of producing a hollow fiber membrane.
Fig. 2 is a schematic view for explaining depth filtration.
Fig. 3 is a schematic view for explaining surface filtration (screen filtration).
Fig. 4 gives SEM images of Example 1; (a) a cross section of a hollow fiber membrane, (b) the inner surface side in (a), and (c) the outer surface side in (a).
Fig. 5 is an SEM image of a cross section of a hollow fiber membrane of Plasmaflo (a reference product).
Fig. 6 is an SEM image of a cross section of a hollow fiber membrane according to Comparative Example 1.
Fig. 7 is a graph for explaining the method for measuring most-frequent pore sizes.
Fig. 8 is a schematic view of a loop-type mini-module.

DESCRIPTION OF EMBODIMENTS

In the following, a description will be given of embodiments of the present invention, but the scope of the present invention is not limited to these embodiments.

<Hollow Fiber Membrane>

(Polyethersulfone)

A hollow fiber membrane according to the present embodiment contains polyethersulfone. The ratio of polyethersulfone to the materials of the hollow fiber membrane is preferably 90 mass% or more, more preferably 93 mass% or more, further preferably 95 mass% or more. The hollow fiber membrane may be solely composed of polyethersulfone.
Polyethersulfone is a compound that contains a structural unit represented by a formula (1) below.

[Chem. 1]

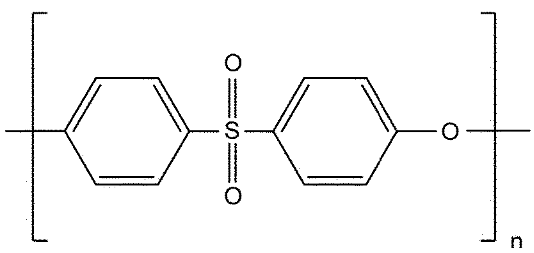

(1)

The polyethersulfone may be, for example, Ultrason (registered trademark) E2020P, E6020P manufactured by BASF, or SUMIKAEXCEL (registered trademark) 3600P, 4100P, 4800P, 5200P, 7600P manufactured by Sumitomo Chemical, or the like, and preferably, it is E6020P, 4800P, or 5200P. These may be used singly or in combination.

(Vinylpyrrolidone-Based Polymer)

The hollow fiber membrane according to the present embodiment may further contain a vinylpyrrolidone-based polymer. The vinylpyrrolidone-based polymer allows for reducing adsorption of blood components such as platelets and proteins to the hollow fiber membrane.
The vinylpyrrolidone-based polymer refers to a polymer composed of monomers that contain at least N-vinylpyrrolidone.
The vinylpyrrolidone-based polymer preferably contains a structural unit represented by a formula (2) below. The vinylpyrrolidone-based polymer may be Kollidon (registered trademark) 30, 90, or Luvitec (registered trademark) K30, K80, K85, K90, VA64, each of which is commercially available from BASF. These may be used singly or in combination.

[Chem. 2]

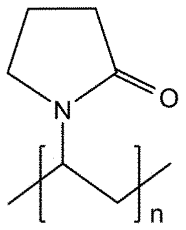

(2)

The content rate of the vinylpyrrolidone-based polymer to the entire hollow fiber membrane is preferably from 1 to 10 mass%, more preferably from 1.5 to 5 mass%.

(Structure etc. of Hollow Fiber Membrane)

The hollow fiber membrane according to the present embodiment has a structure that is non-uniform in a thickness direction (an asymmetric structure). Examples of the hollow fiber membrane having an asymmetric structure include a hollow fiber membrane in which the density, porosity, cross-sectional aperture ratio, and/or the like varies in the thickness direction.

In the hollow fiber membrane having an asymmetric structure according to the present embodiment, the average pore size at the inner surface side in a cross section (a transverse section, a cross section in the thickness direction) is larger than the average pore size at the outer surface side. For example, the hollow fiber membrane according to the present embodiment has a structure which is dense on the outer surface side and less dense on the inner surface side.

In the present embodiment, the gradient index of the hollow fiber membrane is 2.00 or more. The gradient index refers to the ratio of the average pore size at the inner surface side in a cross section of the hollow fiber membrane to the average pore size at the outer surface side. The average pore size and the gradient index can be measured by methods described below in Examples.

Referring to Fig. 2, in plasmapheresis using a hollow fiber membrane 3, from the pushed side (the upper side in Fig. 2; the inner surface side of the hollow fiber membrane), plasma from blood permeates through hollow fiber membrane 3. Among the components of the plasma (such as albumin, fibrinogen, autoimmune antibodies IgG and IgM, and cytokines which can be disease-causing substances), smaller components 51, 52 (such as cytokines) tend to pass through the pores of the hollow fiber membrane, but larger components 4 (such as IgG and IgM), for example, can clog the pores of the hollow fiber membrane. Here, as seen in Fig. 2, in the case of filtration through hollow fiber membrane 3 having an asymmetric structure (depth filtration), components 4 with larger molecular weights disperse in the thickness direction of hollow fiber membrane 3 and adhere thereto (clogging occurs sequentially in the membrane thickness direction). As a result, clogging tends not to occur.

In contrast, as seen in Fig. 3, in the case of filtration through hollow fiber membrane 3 having a structure that is uniform in the thickness direction (surface filtration, screen filtration), components 4 of plasma with larger molecular weights tend to crowd and become deposited near the outermost surface on the pushed side of hollow fiber membrane 3 (the upper side in Fig. 3, the inner surface side of the hollow fiber membrane), and thereby clogging tends to occur.

The most-frequent pore size in a cross section of the hollow fiber membrane according to the present embodiment is preferably 0.090 μm or more. Moreover, the most-frequent pore size is preferably 0.5 μm or less, more preferably 0.3 μm or less.

The most-frequent pore size can be measured by a method described below in Examples.

The inner diameter of the hollow fiber membrane is preferably from 150 μm to 400 μm, more preferably from 200 μm to 350 μm.

The thickness of the hollow fiber membrane is preferably from 50 to 200 μm, more preferably from 60 to 150 μm. The membrane thickness can be calculated by "((Outer diameter)-(Inner diameter))/2".

The hollow fiber membrane (a membrane of the hollow fiber type) is preferably made of a semipermeable membrane.

The hollow rate of the hollow fiber membrane is preferably from 15 to 60%, more preferably from 20 to 50%. The hollow rate is the proportion of the area of hollow portions in a transverse section of the hollow fiber membrane, and it is represented by "(Cross-sectional area of hollow portions)/((Cross-sectional area of membrane portion)+(Cross-sectional area of hollow portions))×100 (%)".

(Purpose of Use)

The hollow fiber membrane according to the present embodiment can be suitably used for separating biological

materials and the like that tend to cause clogging (plasmapheresis, etc.), for example.

In plasmapheresis, plasma is separated from blood. Plasma is the liquid component of blood, a component other than cell components such as blood cells (leucocytes and erythrocytes) and platelets. Plasma contains water, proteins, inorganic salts, sugars, lipids, nitrogen compounds, and the like, and it also contains albumin, fibrinogen, IgG, cytokines, and the like.

<Method of Producing Hollow Fiber Membrane>

In the following, a description will be given of an embodiment of a method of producing the above-mentioned hollow fiber membrane.

A method of producing the hollow fiber membrane according to the present embodiment is, for example, the method described below.

A method of producing the above-mentioned hollow fiber membrane, comprising:

a spinning step that involves discharging a spinning dope and an internal liquid through a double-tube nozzle via an air gap into a coagulation liquid, allowing the spinning dope to become coagulated in the coagulation liquid, and pulling out the coagulated spinning dope from the coagulation liquid to obtain a hollow fiber membrane, wherein
the spinning dope contains a resin raw material containing polyethersulfone, a solvent, and a non-solvent,
the internal liquid contains water,
the total concentration of components of the internal liquid except water (an internal liquid concentration) is from 70 to 90 mass%, and
the nozzle draft ratio is from 1.5 to 6.5.

[Spinning Step]

Referring to Fig. 1, in the spinning step, a spinning dope 10a and an internal liquid 10b are discharged through a double-tube nozzle 11 via an air gap 20 into a coagulation liquid 21, allowing the spinning dope to become coagulated in coagulation liquid 21, and then the coagulated spinning dope is pulled out from coagulation liquid 21 to obtain hollow fiber membrane 3. The pulling-out of the hollow fiber membrane, etc. is performed with the use of in-liquid guides 12, 13 and rollers 14, 15, 16, for example. After pulled out from coagulation liquid 21, hollow fiber membrane 3 is immersed in a water bath 22 and then taken up on a take-up apparatus 23, for example.

Nozzle 11 has a double-tube form, and comprises an outer tube and an inner tube provided on the inner side of the outer tube. The spinning dope is discharged through the gap (a slit) between the outer tube and the inner tube, and the internal liquid is discharged from inside of the inner tube. The inner diameter of the outer tube is preferably from 500 to 1500 μm, more preferably from 600 to 1200 μm. The outer diameter of the inner tube is preferably from 150 to 700 μm, more preferably from 150 to 600 μm. The outer diameter of the inner tube is preferably approximately the same as the inner diameter of the hollow fiber membrane.

When a nozzle of this type is used, it is possible to set the discharge linear speed of the spinning dope and the take-up speed thereof to be approximately the same as each other, which makes it possible to reduce interface friction between the spinning dope and the internal liquid during discharging and, thereby, reducing roughness of the inner face of the hollow fiber membrane.

The discharge linear speed of the spinning dope is determined by dividing the discharged amount of the spinning dope by the slit cross-sectional area, [$\pi(a/2)^2-\pi(b/2)^2$], which is determined from the inner diameter (a) of the outer tube and the outer diameter (b) of the inner tube (see the equation below).

Discharge linear speed of spinning dope [m/min]=(Discharged amount of spinning dope)/(Slit cross-sectional area)

The discharged amount of the spinning dope is preferably from 1.0 to 5.0 cc/min, more preferably from 1.5 to 4.0 cc/min.

The take-up speed is the rotational speed of roller 14 provided at the outlet of the coagulation bath (the surface speed) (see Fig. 1).

The nozzle draft ratio, which is the ratio of the discharge linear speed to the take-up speed ((Take-up speed)/(Discharge linear speed)), is preferably from 1.5 to 6.5, more preferably from 2.0 to 6.0. In this way, by setting the discharge linear speed of the spinning dope higher than the take-up speed, it is possible to achieve the characteristic membrane structure of the hollow fiber according to the present invention.

The straight-line distance of air gap 20 (the distance between the tip of nozzle 11 and the liquid level of coagulation liquid 21) is preferably from 10 to 200 mm, more preferably from 10 to 100 mm.

The hollow fiber membrane obtained by the spinning step may be further subjected to a step of rinsing with pure water (a water rinse step). In the water rinse step, water is made to flow preferably in the opposite direction to the direction of the movement of the hollow fiber membrane (a countercurrent), but it may be made to flow in the same direction as the direction of the movement of the hollow fiber membrane (a parallel current).

(Spinning Dope)

Spinning dope 10a contains the above-mentioned resin raw material containing polyethersulfone, a solvent, and a non-solvent, for example.

The temperature of the spinning dope in nozzle 11 (the set temperature) is preferably from 50 to 110°C, more preferably from 60 to 100°C.

The concentration of polyethersulfone in the spinning dope is preferably from 10 to 30 mass%, more preferably from 10 to 25 mass%. When the concentration of polyethersulfone is too low, the strength of the hollow fiber membrane may become too low. On the other hand, when the concentration of polyethersulfone is too high, the viscosity of the spinning dope may become too high and make it difficult to perform spinning.

When a vinylpyrrolidone-based polymer is added to the spinning dope, the concentration of the vinylpyrrolidone-based polymer in the spinning dope is preferably from 3 to 20 mass%, more preferably from 5 to 15 mass%. When the concentration of the vinylpyrrolidone-based polymer is too low, during plasmapheresis, proteins and the like in the blood tend to become deposited to cause clogging, which may accelerate over-time degradation of performance. On the other hand, when the concentration of the vinylpyrrolidone-based polymer is too high, the viscosity of the spinning dope may become too high and make it difficult to perform spinning.

The solvent is a liquid in which polyethersulfone is soluble. The solvent is preferably a polar solvent, preferably soluble in water. The polar solvent is preferably an aprotic polar solvent. Examples of the aprotic polar solvent include N-methylpyrrolidone (NMP), dimethylformamide (DMF), dimethylacetamide (DMA), dimethyl sulfoxide (DMSO), acetonitrile, and the like.

The non-solvent is a liquid (except water) in which polyethersulfone is insoluble. The non-solvent may be glycol ester, glycerol, and/or alcohol, for example, and it is preferably glycol ester. Examples of the glycol ester include ethylene glycol, triethylene glycol (TEG), polyethylene glycol (such as polyethylene glycol 200 and polyethylene glycol 400), propylene glycol, and the like.

In the spinning dope, the ratio of the amount of the solvent (S) to that of the non-solvent (NS) (the S/NS ratio) is preferably from 35/65 to 55/45, more preferably from 40/60 to 50/50. When the S/NS ratio of the spinning dope is too low, dissolution of polyethersulfone becomes unstable, which may impair spinning stability and/or make it impossible to obtain an asymmetric structure suitable for the purpose of use intended by the present invention. When the S/NS ratio is high, a hollow fiber membrane with an asymmetric structure may not be obtained and/or spinning stability may be impaired.

In addition to the solvent and the non-solvent, the spinning dope may further contain water.

At the time of mixing the resin raw material containing polyethersulfone which is the material of the hollow fiber membrane, the solvent, and the non-solvent, the order to add the materials and the method for mixing them are not particularly limited.

(Internal Liquid)

The internal liquid contains water. The content rate of water in the internal liquid is preferably from 10 to 30 mass%, more preferably from 15 to 25 mass%.

In addition to water, the internal liquid may contain a solvent, a non-solvent, and/or the like. Examples of the non-solvent include ethylene glycol, triethylene glycol (TEG), polyethylene glycol 200 or 400, glycerol, propylene glycol, and the like. Examples of the solvent include N-methylpyrrolidone (NMP), dimethylacetamide, dimethylformamide, dimethyl sulfoxide, and the like.

For obtaining the hollow fiber membrane according to the present embodiment that has an asymmetric structure in which the average pore size at the inner surface side is relatively large and the average pore size at the outer surface side is relatively small, it is preferable to set the concentration of the components, except water, contained in the internal liquid (such as the solvent and/or the non-solvent) (the internal liquid concentration ratio) to be higher than the concentration of the coagulation liquid. The internal liquid concentration is preferably from 70 to 90 mass%, more preferably from 75 to 85 mass%.

Moreover, for obtaining the hollow fiber membrane according to the present embodiment, it is preferable to adjust the nozzle draft ratio to fall within a particular range. The nozzle draft ratio is preferably from 1.5 to 6.5, more preferably from 2.0 to 6.0.

At the time of discharging spinning dope 10a and internal liquid 10b through double-tube nozzle 11, the temperature of the spinning dope may be set to be different from that of the internal liquid.

(Coagulation Liquid)

The coagulation liquid preferably contains a solvent and a non-solvent (except water). In that case, in addition to the solvent and the non-solvent, the coagulation liquid may further contain water.
The total proportion of the solvent and the non-solvent in the coagulation liquid (the concentration of the coagulation liquid, the CB concentration) is preferably from 60 to 90 mass%, more preferably from 65 to 90 mass%.
The ratio of the amount of the solvent to that of the non-solvent in the coagulation liquid is from 40/60 to 50/50, for example.
Moreover, the temperature of the coagulation liquid is preferably from 30 to 80°C, more preferably from 40 to 70°C.

[Examples]

In the following, a more detailed description will be given of the present invention by way of Examples, but these Examples are not intended to limit the scope of the present invention.

[Example 1]

Basically in the same manner as the method of producing a hollow fiber membrane described in EMBODIMENTS, a hollow fiber membrane according to Example 1 was produced. Specific conditions and the like are as follows.

(Preparation of Spinning Dope)

17.0 mass% of PES (manufactured by BASF, 6020P), 10.0 mass% of PVP (manufactured by BASF, Kollidon (registered trademark) K30), 32.85 mass% of N-methylpyrrolidone (NMP, manufactured by Mitsubishi Chemical, a solvent), and 40.15 mass% of triethylene glycol (TEG, manufactured by Mitsubishi Chemical, a non-solvent) were mixed and dissolved to obtain a spinning dope.

(Composition of Internal Liquid)

A mixed liquid of 37.8 mass% of NMP (solvent), 46.2 mass% of TEG (non-solvent), and 16.0 mass% of reverse osmosis (RO) water. The total concentration of the solvent and the non-solvent in the internal liquid (IL) (the internal liquid concentration, the IL concentration) is 84 mass%.

(Composition of Coagulation Liquid)

A mixed liquid of 26.1 mass% of NMP (solvent), 31.9 mass% of TEG (non-solvent), and 42.0 mass% of RO water. The total concentration of the solvent and the non-solvent in the coagulation liquid (CB) (the CB concentration) is 58 mass%.

(Conditions in Spinning Step)

Spinning dope discharge temperature (the set temperature): 67°C
Air gap length (AG length): 30 mm
Pull-out speed (spinning rate): 15 m/min (to wind on a reel (take-up apparatus 23))
Nozzle draft ratio: 4.05

(Conditions in Water Rinse Step)

Flow in water tank (water bath): Countercurrent
Temperature: 30°C

A bundle of the hollow fiber membranes obtained by the above-mentioned steps was cut into a certain length, wrapped in gauze, and then rinsed with RO water at 80°C, followed by drying at 60°C with a hot-air dryer.

In this way, dried hollow fiber membranes were obtained. The inner diameter of the hollow fiber membranes thus obtained (the membrane inner diameter) was 300 μm, and the thickness thereof (the membrane thickness) was 75 μm.

(Measurement of Inner Diameter and Membrane Thickness of Hollow Fiber Membrane)

The membrane inner diameter and the membrane thickness were measured by the methods described below.
A proper number of the hollow fiber membranes are drawn through a hole (diameter, 3 mm) that is made in the center of a slide glass, in a way that the hollow fiber membranes will not slide out of the hole, followed by cutting the hollow fiber membranes with a razor along the top face and the bottom face of the slide glass to obtain hollow fiber membrane cross section samples. For the hollow fiber membrane cross section samples thus obtained, with the use of a projector (Nikon PROFILE PROJECTOR V-12), the inner diameter and the outer diameter of the hollow fiber membranes are measured.
More specifically, for one hollow fiber membrane cross section, the dimensions of the outer surface of the hollow fiber membrane in both the X-X direction and the Y-Y direction (two mutually-orthogonal directions in the cross section) are measured, and the arithmetic mean of them is defined as the outer diameter of the one hollow fiber membrane cross section. Similarly, for one hollow fiber membrane cross section, the dimensions of the hollow portion in both the X-X direction and the Y-Y direction (two mutually-orthogonal directions in the cross section) are measured, and the arithmetic mean of them is defined as the inner diameter of the one hollow fiber membrane cross section. This measurement is carried out for ten cross sections, and the average values are defined as the inner diameter and the outer diameter, respectively.
The membrane thickness (the average value) is calculated by "((Outer diameter)-(Inner diameter))/2", based on the results of measurement of the inner diameter and the inner diameter of each hollow fiber membrane (the average values).

[Examples 2 to 5, Comparative Example 1]

Production conditions and the like for the hollow fiber membrane were changed as specified in Table 1. Except for these changes, the same manner as in Example 1 was adopted to produce hollow fiber membranes according to Examples 2 to 5 and Comparative Example 1. In Table 1, "%" represents "mass%".
In Fig. 6, an SEM image of a cross section of the hollow fiber membrane according to Comparative Example 1 is shown.

<Evaluation of Performance of Hollow Fiber Membrane>

For each of the hollow fiber membranes according to Examples and Comparative Example, the following measurement was carried out. Results of the measurement are shown in Table 2. Moreover, a scanning electron microscope (SEM) image of the hollow fiber membrane according to Example 1 is shown in Fig. 4.

(Preparation of Module)

A bundle of a plurality of hollow fiber membranes, which were cut into a length of about 30 cm, was wrapped with a polyethylene film, and thereby a hollow fiber membrane bundle was obtained. This hollow fiber membrane bundle was inserted into a tubular polycarbonate vessel having a round tubular shape, and both ends were hardened with a urethane potting agent. The end portions were cut off, and thereby a module that was open at both ends was obtained. The number of the hollow fiber membranes was determined as appropriate. At two positions on the round tubular face of the tubular vessel having a round tubular shape, ports were provided for allowing fluid perfusion outside the hollow fiber membranes, and, also, to both ends of the tubular vessel, headers were attached for allowing fluid perfusion inside the hollow fiber membranes.

(Preparation of Loop-Type Mini-Module)

A bundle of a plurality of hollow fiber membranes, which were cut into a length of about 40 cm, was bent in such a manner that the end portions were aligned side by side, and the end portions were inserted into a pipe (a sleeve) and hardened with a urethane potting agent. The bonded portion was cut off partially, and thereby a loop-type mini-module was obtained in which the hollow portion of each hollow fiber membrane was open (Fig. 8). The number of the hollow fiber membranes was determined as appropriate.
For reference purposes, the same measurement was carried out for Plasmaflo (manufactured by Asahi Kasei Medical Co., Ltd.), a commercially available membrane-type plasmapheresis device equipped with hollow fiber membranes. Results of the measurement are also shown in Table 2. The hollow fiber membrane used in Plasmaflo is a hollow fiber membrane made of hydrophobic polyethylene, and the surface (the outer surface) is coated with ethylene vinyl alcohol copolymer. The inner diameter of the hollow fiber membrane is 250 μm, and the thickness thereof is 50 μm. A scanning electron microscope (SEM) image of a hollow fiber membrane used in Plasmaflo is shown in Fig. 5.

[Measurement of Gradient Index]

Firstly, an image of a cross section of each hollow fiber membrane (see Fig. 4(a)) is captured with the use of a scanning electron microscope (SEM) at a magnification of 10000 times. In the cross-sectional image thus captured, a measurement region was defined for each of the inner surface side of the hollow fiber membrane (see Fig. 4(b)) and the outer surface side thereof (see Fig. 4(c)), which stretched 5 μm in the membrane thickness direction (from the inner surface) and 10 μm in the direction perpendicular to the membrane thickness direction. With the use of an image analysis software, binarization of the measurement region was performed to acquire a black-and-white image, and pore size distribution was measured for both the inner surface side and the outer surface side of the hollow fiber membrane in the cross section to determine the equivalent circle diameter. The average value of the equivalent circle diameters thus obtained was defined as the average pore size.

The threshold value was increased sequentially by five percentage points, and at each threshold value, pores were counted. Based on the results, the threshold value that yielded the greatest pore count was identified. Based on the threshold value thus identified, the above-mentioned binarization was carried out.

The gradient index was calculated from the value of the average pore size at the inner surface side (see Fig. 4(b)) and the outer surface side (see Fig. 4(c)) in cross sections of the hollow fiber membranes (see Fig. 4(a)), by the following equation.

Gradient index=(Average pore size at inner surface side in cross sections of hollow fiber membranes)/(Average pore size at outer surface side in cross sections of hollow fiber membranes)

[Measurement of Most-Frequent Pore Size]

As for the loop-type mini-module, with the use of a perm porometer (a through-pore size distribution measurement apparatus) manufactured by PMI, the average pore size was measured. As the solvent for the perm porometer, perfluoropolyester (under the trade name of "Galwick") was used. From the results of the average pore size measurement thus obtained, average pore size distribution was determined, which is given in Fig. 7, and the average pore size value with the highest distribution (with the highest pore distribution density) was defined as the most-frequent pore size (see Fig. 7).

[Measurement of Largest Pore Size]

The loop-type mini-module was completely immersed in a sufficient amount of 2-propanol (hereinafter abbreviated as iPA) for 3 minutes to let iPA permeate throughout the inner hollow parts and the membrane wall parts. With the entire hollow fiber membrane portion immersed in iPA, the loop-type mini-module was connected to a nitrogen line whose sleeve was equipped with a pressure gauge for allowing monitoring of the applied pressure, followed by pressure application. At the time point when air bubbles started to come out constantly from the membrane wall portions of the hollow fiber membranes, the point was recorded as the bubble point P [Pa]. The measurement was carried out three times for each sample, and the average of the measured values of the bubble point was defined as the bubble point of the sample. Further, by the following equation, the largest pore size was calculated from the bubble point (P [bar]) measured with iPA.

$$\text{Largest pore size } [\mu m] = 4\gamma COS\theta / P$$

γ represents surface tension (N/m) of the solvent, θ represents the contact angle (°) between the membrane material and the solvent, and P represents the bubble point pressure (Pa). Based on the iPA surface tension γ being 20.8, and the contact angle being 0° and thereby COSθ=1, the calculation was performed.

[Measurement of Transmembrane Pressure (TMP)]

Bovine blood which contained citric acid added thereto for coagulation inhibition and which had been adjusted to a hematocrit concentration of 40±2% and a protein concentration of 6 to 7 g/dL was circulated for 180 minutes under conditions of a blood flow rate (Qb) of 100 mL/min and a filtration flow rate (Qf) of 30 mL/min. After 30 minutes from the start of the circulation and after 180 minutes from the start of the circulation, the inlet pressure, the outlet pressure, and the

filtrate pressure were measured. From the measured pressure values thus obtained, transmembrane pressure (TMP) was determined by the following equation.

$$TMP=([Inlet\ pressure]+[Outlet\ pressure])/2-[Filtrate\ pressure]$$

[Measurement of Fibrinogen Sieving Coefficient (SCFib)]

The same test as in the above-described TMP test was carried out and, after 30 minutes from the start of the circulation and after 180 minutes from the start of the circulation, liquid was sampled at both the inlet and the outlet and also from the filtrate, and the concentration was determined with an ELISA-KIT (manufactured by Cosmo Bio, Fibrinogen ELISA Kit 96test (Cloud-Clone Corp.)). From the measured values thus obtained, the fibrinogen sieving coefficient (SCFib) was calculated by the following equation.

$$SCFib=2\times[Filtrate\ concentration]/([Inlet\ concentration]+[Outlet\ concentration])$$

Retention of fibrinogen sieving coefficient (SCFib) is an index of the change between 30 minutes after the start of the circulation and 180 minutes after the start of the circulation, and can be calculated by (SCFib after 30 minutes from start of circulation)/(SCFib after 180 minutes from start of circulation). For example, when 100% of fibrinogen permeates through the membrane, the fibrinogen sieving coefficient is 1.

[Table 1]

| Target value | Membrane raw material | Membrane inner diameter (μm) | Membrane thickness (μm) | Nozzle draft ratio | IL concentration (%) | CB concentration (%) | AG length (mm) | Spinning rate (m/min) |
|---|---|---|---|---|---|---|---|---|
| Ex. 1 | PES/PVP | 300 | 75 | 4.05 | 84 | 58 | 30 | 15 |
| Ex. 2 | PES/PVP | 300 | 90 | 3.57 | 84 | 58 | 100 | 15 |
| Ex. 3 | PES/PVP | 300 | 110 | 2.66 | 84 | 58 | 100 | 15 |
| Ex. 4 | PES/PVP | 250 | 75 | 5.54 | 84 | 58 | 40 | 25 |
| Ex. 5 | PES/PVP | 300 | 75 | 4.05 | 80 | 68 | 100 | 15 |
| Comp. Ex.1 | PES/PVP | 300 | 90 | 1.18 | 84 | 58 | 100 | 15 |

[Table 2]

| | Structure of hollow fiber membrane | | | | | Performance | | | | |
|---|---|---|---|---|---|---|---|---|---|---|
| | **Gradient index** | Average pore size [μm] | | **Most-frequent pore size** [μm] | Largest pore size [μm] | Transmembrane pressure (mmHg) | | Fibrinogen sieving coefficient (SCFib) | | |
| | | Inner surface side | Outer surface side | | | 30 min after circulation start | 180 min after circulation start | 30 min after circulation start | 180 min after circulation start | SCFib retention |
| Target value | ≥**2.00** | - | - | ≥**0.090** | - | ≤50 | | >0.90 | | ≥90% |
| Ex. 1 | **2.22** | 0.28 | 0.13 | **0.100** | 0.23 | 15.5 | **43.5** | 1.0 | **0.98** | 98% |
| Ex. 2 | **2.44** | 0.29 | 0.12 | **0.104** | 0.27 | 14.5 | **36.5** | 1.0 | **1.0** | 100% |
| Ex. 3 | **2.23** | 0.31 | 0.14 | **0.116** | 0.28 | 20.0 | **19.0** | 1.0 | **1.0** | 100% |
| Ex. 4 | **2.03** | 0.24 | 0.15 | **0.091** | 0.20 | 34.5 | **23.0** | 0.84 | **0.77** | 92% |
| Ex. 5 | **2.17** | 0.28 | 0.13 | <0.076 | 0.24 | 7.4 | **6.3** | 0.17 | **0.15** | 88% |
| Comp. Ex.1 | 1.70 | 0.24 | 0.14 | **0.081** | 0.28 | 15.5 | **58.0** | 1.0 | **0.68** | 68% |
| Plasmaflo | 1.00 | - | - | **0.187** | 0.68 | 183 | **171** | 1.0 | **1.0** | 100% |

Referring to the results given in Table 2, it is indicated that an elevation of transmembrane pressure (TMP) was reduced to 50 mmHg or less in the hollow fiber membrane according to Examples 1 to 5 where the gradient index was 2.00 or more, as compared to the hollow fiber membrane according to Comparative Example 1 where the gradient index was less than 2.00. It is known that hemolysis tends to occur in plasmapheresis when TMP exceeds 50 mmHg, so in treatment and the like using plasmapheresis, it is important to maintain TMP at 50 mmHg or less.

Because inhibition of TMP elevation occurs mainly due to clogging of hollow fiber membranes, it is conceivable that clogging is reduced in the hollow fiber membrane according to Examples 1 to 5. Accordingly, retention of fibrinogen sieving coefficient (SCFib) is relatively low in the hollow fiber membrane according to Comparative Example 1 which is conceivable to be susceptible to clogging, and retention of SCFib is relatively high in the hollow fiber membrane according to Examples 1 to 5.

Moreover, in the hollow fiber membrane according to Example 5 which has a gradient index of 2.00 or more and a most-frequent pore size of less than 0.090 μm, the fibrinogen sieving coefficient greatly drops after the start. In contrast, in the hollow fiber membrane according to Examples 1 to 4 which has a gradient index of 2.00 or more and a most-frequent pore size of 0.090 μm or more, a substantial decrease of the fibrinogen sieving coefficient was not observed. Moreover, also in the hollow fiber membrane according to Comparative Example 1 which has a gradient index of less than 2.00 and a most-frequent pore size of less than 0.090 μm, a decrease of the fibrinogen sieving coefficient was not observed at the start. From these results, it is conceivable that in a hollow fiber membrane with a high gradient index (2.00 or more), if the most-frequent pore size is less than 0.090 μm, the fibrinogen sieving coefficient tends to be greatly decreased, and from the viewpoint of maintaining the fibrinogen sieving coefficient of the hollow fiber membrane according to the present invention, it is desirable to maintain the most-frequent pore size at 0.090 μm or more.

REFERENCE SIGNS LIST

10a spinning dope, 10b internal liquid, 11 nozzle, 12, 13 in-liquid guide, 14, 15, 16 roller, 20 air gap, 21 coagulation liquid, 22 water bath, 23 take-up apparatus, 3 hollow fiber membrane, 4 larger component, 51, 52 smaller component.

## Claims

**1.** A hollow fiber membrane comprising polyethersulfone, wherein

the hollow fiber membrane has a structure that is non-uniform in a thickness direction, and
a gradient index which is a ratio of an average pore size at an inner surface side in a cross section of the hollow fiber membrane to an average pore size at an outer surface side in the cross section is 2.00 or more.

**2.** The hollow fiber membrane according to claim 1, wherein a most-frequent pore size in a cross section of the hollow fiber membrane is 0.090 μm or more.

**3.** The hollow fiber membrane according to claim 1 or 2 further comprising a vinylpyrrolidone-based polymer.

**4.** The hollow fiber membrane according to any one of claims 1 to 3 for use in plasmapheresis.

**5.** A plasmapheresis method using the hollow fiber membrane according to any one of claims 1 to 4.

FIG.1

SPINNING STEP

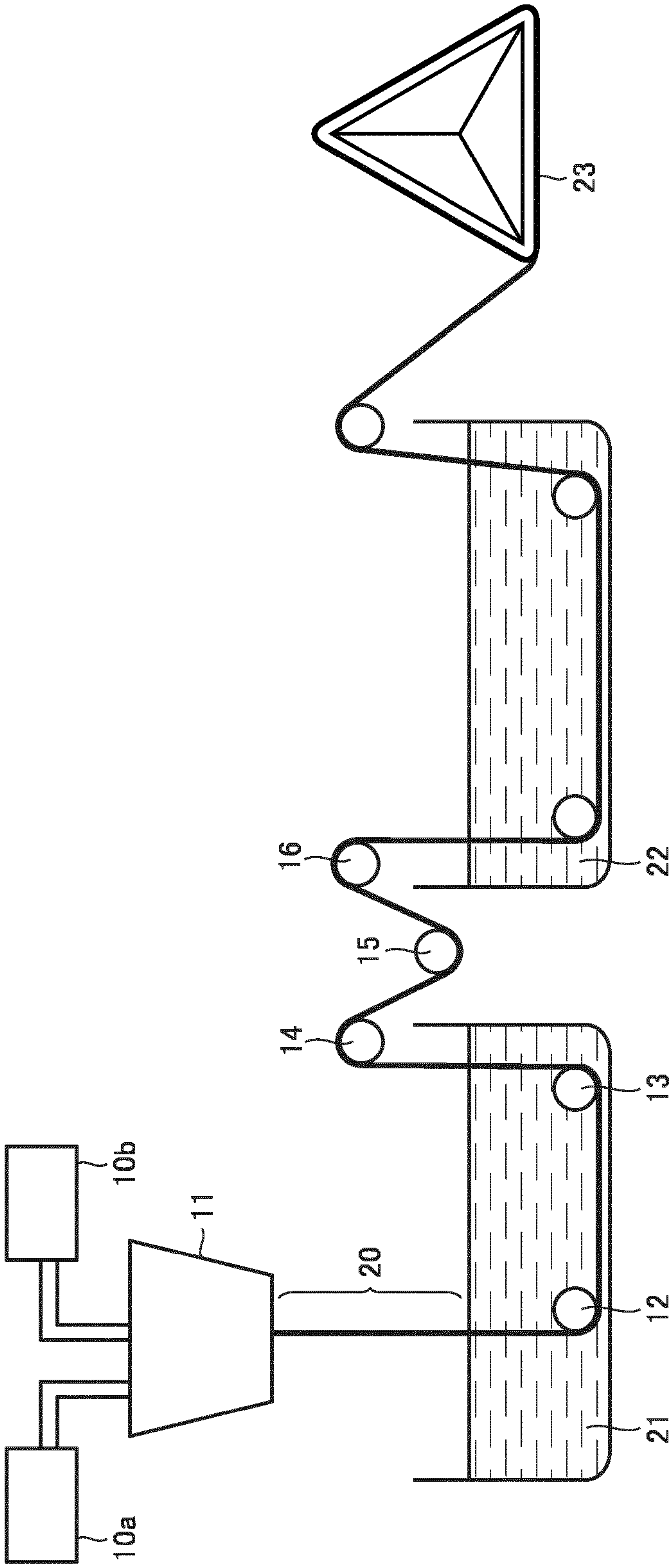

FIG.2

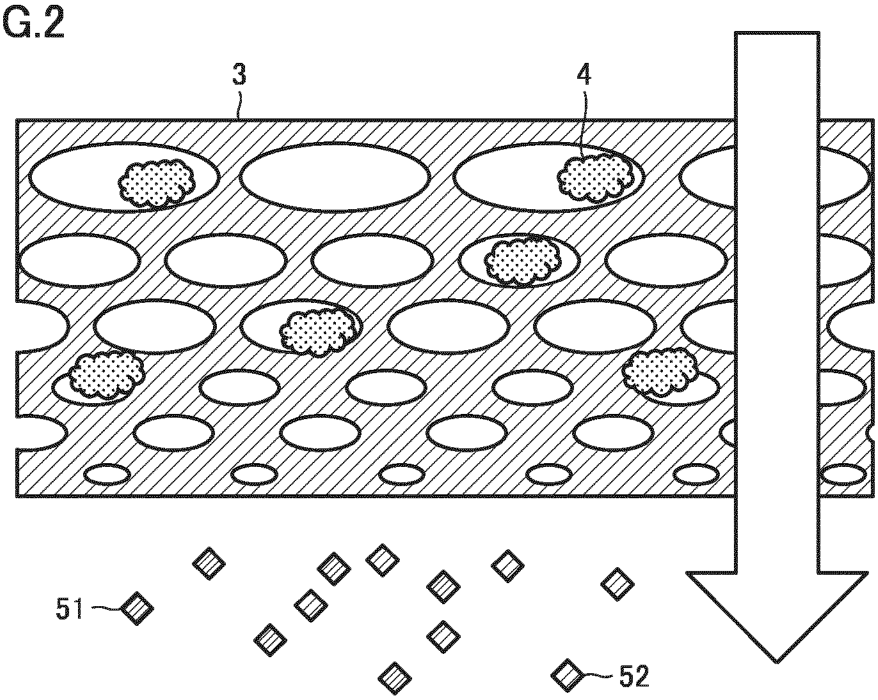

FIG.3

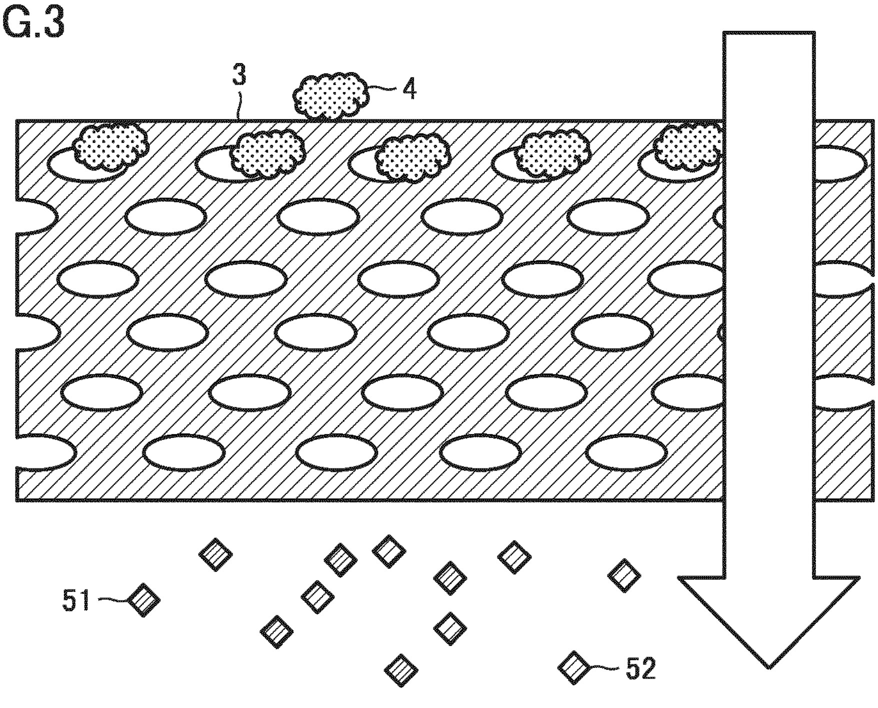

FIG.4

(a)

CROSS SECTION

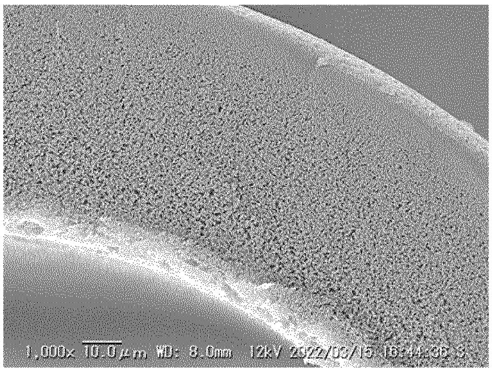

(b) CLOSE-UP VIEW OF INNER SURFACE SIDE IN (a)

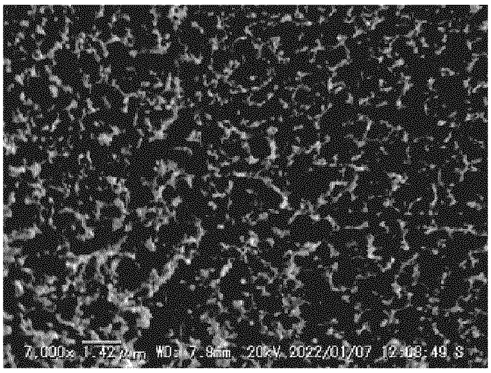

(c) CLOSE-UP VIEW OF OUTER SURFACE SIDE IN (a)

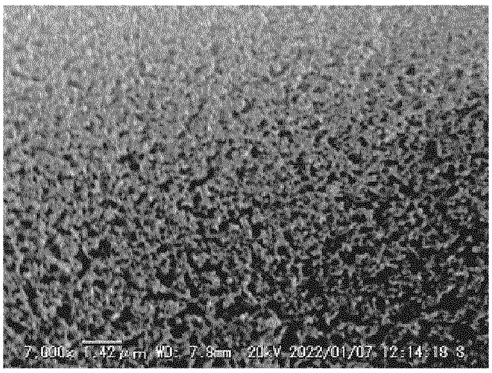

FIG.5

CROSS SECTION

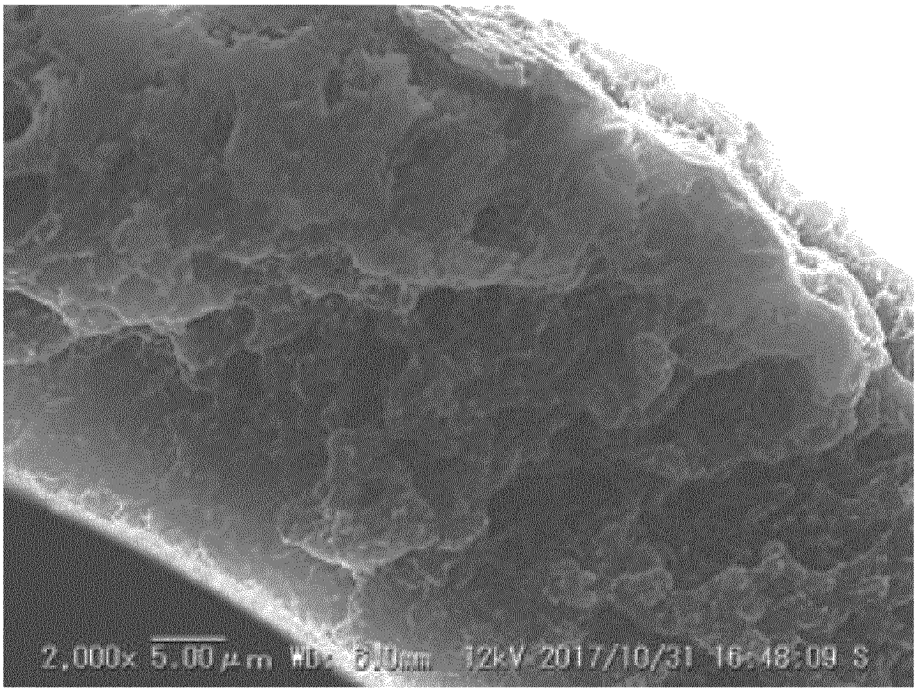

FIG.6

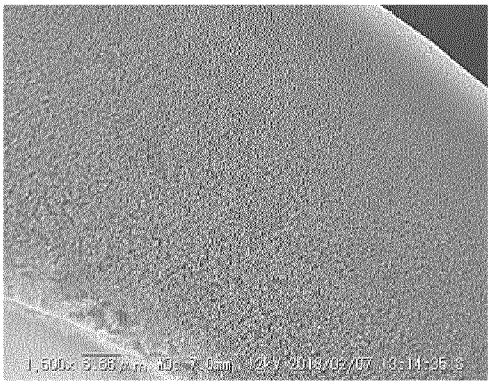

FIG.7

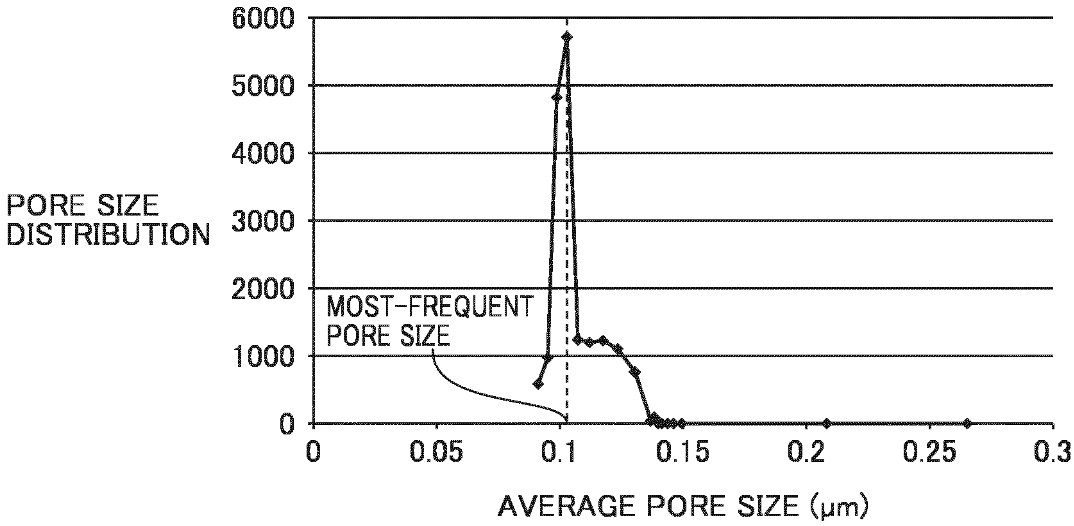
6000
5000
4000
3000
2000
1000
0
PORE SIZE
DISTRIBUTION
MOST-FREQUENT
PORE SIZE
0
0.05
0.1
0.15
0.2
0.25
0.3
AVERAGE PORE SIZE (μm)

FIG.8

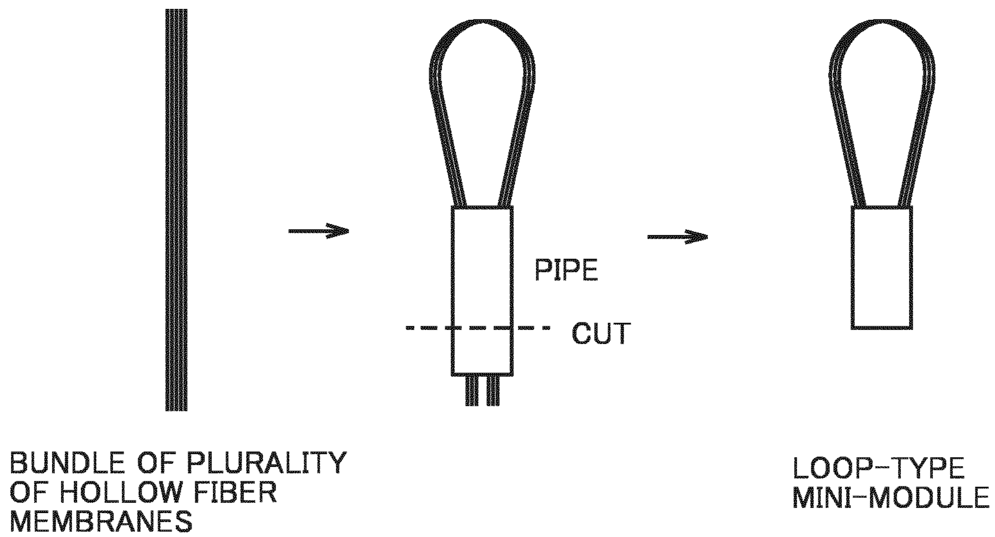
PIPE
CUT
BUNDLE OF PLURALITY OF HOLLOW FIBER MEMBRANES
LOOP-TYPE MINI-MODULE

## INTERNATIONAL SEARCH REPORT

International application No.
**PCT/JP2023/026206**

### A. CLASSIFICATION OF SUBJECT MATTER

***B01D 69/00***(2006.01)i; ***B01D 69/08***(2006.01)i; ***B01D 71/44***(2006.01)i; ***B01D 71/68***(2006.01)i; ***A61M 1/16***(2006.01)i
FI: B01D71/68; A61M1/16 101; B01D69/08; B01D69/00; B01D71/44

According to International Patent Classification (IPC) or to both national classification and IPC

### B. FIELDS SEARCHED

Minimum documentation searched (classification system followed by classification symbols)

B01D69/00; B01D69/08; B01D71/44; B01D71/68; A61M1/16

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | WO 2020/203716 A1 (ASAHI KASEI MEDICAL CO., LTD.) 08 October 2020 (2020-10-08) claims, paragraphs [0002]-[0005], [0009], [0011], [0015], [0020]-[0022], [0029], [0032], [0036], [0043]-[0054], [0070]-[0116], [0127]-[0146] | 1-5 |
| X | WO 2016/031834 A1 (ASAHI KASEI MEDICAL CO., LTD.) 03 March 2016 (2016-03-03) claims, paragraphs [0002]-[0005], [0017], [0019]-[0023], [0030], [0034], [0036]-[0043], [0048]-[0049], [0051], [0055]-[0126], fig. 1 | 1-5 |
| X | JP 2020-121299 A (ASAHI KASEI MEDICAL CO., LTD.) 13 August 2020 (2020-08-13) claims, paragraphs [0001]-[0002], [0043]-[0048], [0053]-[0069], [0087]-[0102] | 1-5 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **11 September 2023** | **26 September 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**
**Information on patent family members**

International application No.
**PCT/JP2023/026206**

| Patent document cited in search report | Publication date (day/month/year) | Patent family member(s) | Publication date (day/month/year) |
|---|---|---|---|
| WO 2020/203716 A1 | 08 October 2020 | US 2022/0168694 A1<br>claims, paragraphs [0002]-[0006], [0023], [0027], [0033], [0049], [0054], [0061]-[0065], [0076]-[0089], [0116]-[0187], [0205]-[0228]<br>EP 3950103 A1<br>KR 10-2021-0126759 A<br>CN 113646067 A | |
| WO 2016/031834 A1 | 03 March 2016 | US 2017/0266626 A1<br>claims, paragraphs [0002]-[0007], [0055], [0057]-[0062], [0078]-[0079], [0090]-[0091], [0098]-[0116], [0123]-[0127], [0132]-[0133], [0141]-[0260], fig. 1<br>EP 3195922 A1<br>KR 10-2016-0137628 A<br>CN 106573203 A | |
| JP 2020-121299 A | 13 August 2020 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 5290168 B
- WO 2020203716 A